# EUROPÄISCHE PATENTSCHRIFT

(11) **EP 2 178 853 B1**
(45) Veröffentlichungstag und Bekanntmachung des Hinweises auf die Patenterteilung: **19.01.2011**
(21) Anmeldenummer: 08787133.1
(22) Anmeldetag: 12.08.2008
(51) Int. Cl.: C07D 301/12

(54) **EFFIZIENTES VERFAHREN ZUR HERSTELLUNG VON EPOXIDEN DURCH OXIDATION VON OLEFINEN IN DER HOMOGENEN GASPHASE**
EFFICIENT PROCESS FOR PRODUCING EPOXIDES BY OXIDATION OF OLEFINS IN THE HOMOGENEOUS GAS PHASE
PROCÉDÉ EFFICACE DE PRODUCTION D'ÉPOXYDES PAR OXYDATION D'OLÉFINES EN PHASE GAZEUSE HOMOGÈNE

(30) Priorität: 20.08.2007 DE 102007039874
(43) Veröffentlichungstag der Anmeldung: 28.04.2010
(73) Patentinhaber: Zylum Beteiligungsgesellschaft Mbh &Co. Patente II, 12529 Schönefeld / Waltersdorf (DE)
(72) Erfinder: BERNDT, Torsten, 37327 Leinefelde (DE)
(74) Vertreter: Zech, Stefan Markus
(86) Internationale Anmeldenummer: PCT/EP2008/060571
(87) Internationale Veröffentlichungsnummer: WO 2009/024503

(56) Entgegenhaltungen:
- WO-A-02/20502
- BERNDT T ET AL: "APPLIED CHEMISTRY:GAS-PHASE EPOXIDATION OF PROPYLENE AND ETHYLENE" INDUSTRIAL & ENGINEERING CHEMISTRY RESEARCH, AMERICAN CHEMICAL SOCIETY, US, Bd. 44, 1. Januar 2005 (2005-01-01), Seiten 645-650, XP002446824 ISSN: 0888-5885 in der Anmeldung erwähnt

## Beschreibung

Die Erfindung betrifft ein wirtschaftliches Einstufenverfahren zur Herstellung von Epoxiden durch Oxidation von Olefinen in einer homogenen Gasphasenreaktion, indem das Olefin in einem Strömungsreaktor mit einer Gasmischung aus Ozon und NO₂ und/oder NO als Oxidationsmittel ohne Verwendung eines Katalysators umgesetzt wird, wobei Ozon und NO₂ und/oder NO in einer dem Strömungsreaktor vorgeschalteten Mischkammer gemischt werden, dass dadurch gekennzeichnet ist, dass das Olefin in der Reaktionszone des Strömungsreaktors bei einer Reaktionstemperatur von etwa 150 °C bis etwa 450 °C und einem Druck von 250 mbar bis 10 bar mit der Gasmischung des Oxidationsmittels umgesetzt wird, wobei der das Olefin enthaltende Trägergasstrom in einer Vorheizzone des Strömungsreaktors auf eine Temperatur von 250 °C bis 650 °C erhitzt wird, die Gasmischung des Oxidationsmittels aus der Mischkammer mit Umgebungstemperatur dem Olefin in der Reaktionszone des Strömungsreaktors turbulent zugemischt wird, so dass die Reaktionstemperatur beim Zumischen erreicht wird, und das Verhältnis zwischen Olefin-Gasstrom und Gasstrom des Oxidationsmittels von 5 : 1 bis 1 : 1 beträgt.

Es ist bekannt, Epoxide durch Oxidation von Olefinen in einer homogenen Gasphasenreaktion herzustellen, indem ein Ozon/NOₓ-Gasstrom als Oxidationsmittel dient und die Umsetzung bei milden Reaktionsbedingungen ohne Verwendung eines Katalysators erfolgt. So wird in WO 02/20502 A1 in den Beispielen die Oxidation von Propylen, trans-Butylen und iso-Butylen bei Drücken von 10 und 25 mbar und Temperaturen zwischen 140 - 230 °C beschrieben. Die erzielten Selektivitäten' an erzeugtem Epoxid liegen zwischen 68,9 und 96,9 %.

In Ind. Eng. Chem. Res. 2005, 44, S. 645 - 650 beschreiben Berndt, T. und Böge O. weitere Untersuchungen zur Gasphasenepoxidierung von Propylen und Ethylen. Propylenoxid und Ethylenoxid sind wirtschaftlich interessante Epoxide, da sie Vorstufen zur Produktion von Polymeren (Polyester, Polyurethane) oder Lösungsmitteln (Glycole) darstellen. Die Untersuchungen der genannten Publikation zeigen einerseits, dass bei einem steigenden Druck von 25, 50, 100 und 200 mbar (Temperatur 300 °C) die Selektivität zum Propylenoxid deutlich von 89,1 % auf 56,6 % abfällt (vgl. S. 646, linke Spalte, "Results and Discussion"). Andererseits ergaben die Untersuchungen, dass sich das molare Verhältnis von umgesetztem Propylen zu eingesetztem Ozon (Ozonausnutzung Δ [C₃H₆] / [O₃]₀) ebenfalls mit steigendem Druck verschlechtert (vgl, S. 648, Tabelle 3).

Zur technischen Realisierung eines effizienten industriellen Prozesses sind jedoch Drücke, die weit unter Normaldruck liegen, ungeeignet, da dann eine höhere Pumpkapazität bereitzustellen ist, was sich negativ auf die Investitions- und Energiekosten auswirkt. Trotz höherer Drücke sollte die Selektivität zum Epoxid in einem industriellen Prozess mindestens 80 % betragen und insbesondere das molare Verhältnis von umgesetztem Epoxid zu eingesetztem Ozon möglichst den Wert 1 erreichen (d. h., eine Ozonausnutzung von 100 %), da Ozon kostenintensiv ist.

Diese Aufgabe wird gemäß Anspruch 1 der vorliegenden Erfindung gelöst. Die Unteransprüche stellen bevorzugte Ausführungsformen dar.

Es wurde überraschenderweise gefunden, dass trotz höherer Drücke von 250 mbar bis 10 bar, insbesondere bei Drücken von 500 bis 2000 mbar, vorzugsweise bei mehr als 1000 mbar, besonders bevorzugt bei Normaldruck, molare Verhältnisse von umgesetztem Epoxid zu eingesetztem Ozon erreicht werden, die nahezu 1 und sogar größer 1 sind, wenn nach den in Anspruch 1 genannten Bedingungen verfahren wird. Dieser überraschende Befund, dass mehr Olefin umgesetzt wird als Ozon eingesetzt wird, ist gegenwärtig mechanistisch unklar. Mit den in Anspruch 1 genannten Bedingungen werden auch gute Selektivitäten von größer 80 % und teilweise über 90 % erreicht.

Gemäß der Erfindung wird der das Olefin enthaltende Trägergasstrom auf eine Temperatur von 250 bis 650 °C, die höher als die eigentliche Reaktionstemperatur ist, vorgeheizt. Vorzugsweise wird der Olefin-Gasstrom auf 400 bis 550 °C vorgeheizt. Dies erfolgt in der Vorheizzone des Strömungsreaktors. Der Gasstrom aus Ozon und NO₂ und / oder NO und ggf. Trägergas, der in der Mischkammer gemischt wird, wird mit Raumtemperatur (18 bis 25 °C) der Reaktionszone des Strömungsreaktors (vorzugsweise stromabwärts am Beginn der Reaktionszone) turbulent zugemischt, so dass (mindestens) die Reaktionstemperatur sofort erreicht wird, wobei "sofort" heißt, dass die Reaktionstemperatur innerhalb der ersten 5 bis 10 % der Verweilzeit in der Reaktionszone erreicht wird. Die Reaktionstemperatur beträgt von etwa 150 bis etwa 450 °C, vorzugsweise von etwa 200 °C bis etwa 350 °C.

"Turbulentes" Mischen bedeutet im Sinne der vorliegenden Erfindung z. B. das Einbringen des Gasstromes des Oxidationsmittels Ober Düsen, über der Einbau von Gittern oder das Arbeiten mit einem turbulenten Freistrahl oder andere geeignete Herangehensweisen. Auf jeden Fall soll mit dieser Maßnahme eine quasi momentane, ideale Duschmischung erreicht werden.

Erfindungsgemäß ist auch das Verhältnis zwischen Olefin-Gasstrom und Gasstrom des Oxidationsmittels so gewählt, dass nach dem turbulenten Mischen die Reaktionstemperatur erreicht ist. Das Verhältnis zwischen Ofefin-Gasstrom und Gasstrom des Oxidationsmittels beträgt von 5 : 1 bis 1 : 1, vorzugsweise von 4 : 1 bis 2 : 1.

Die Verweilzeiten in der Reaktionszone betragen von 1 ms bis maximal einige Sekunden. Vorzugsweise liegen sie zwischen 1 ms und 250 ms.

Gemäß der Erfindung wird Ozon vorzugsweise als Ozon/Sauerstoffgemisch eingesetzt, insbesondere mit 1 - 15 Vol.-% Ozon im Sauerstoff, ganz besonders bevorzugt von 5-10 Vol.-% Ozon im Sauerstoff. Es werden Ozon und NO₂ in einem Verhältnis kleiner 0.5 eingesetzt. Ozon und NO werden vorzugsweise in einem Verhältnis kleiner 1.5 eingesetzt.

Als Trägergas für das Olefin und für das Gasgemisch des Oxidationsmittels wird entweder ein Inertgas eingesetzt, z. B. Helium, Argon oder Stickstoff, Luft oder Sauerstoff oder Gemische der genannten Gase. Vorzugsweise wird Stickstoff angewendet.

Das erfindungsgemäße Verfahren wird in einem Strömungsreaktor durchgeführt, wie er prinzipiell in WO 02/20502 A1 beschrieben ist. Der Strömungsreaktor der vorliegenden Erfindung weist lediglich neben der Reaktionszone noch zusätzlich eine Vorheizzone zum Vorheizen des Olefin-Gasstromes auf, die sich bis zum Beginn der Reaktionszone erstreckt und sich an diese ohne Unterbrechung direkt anschließt und separat von der Reaktionszone beheizt wird.

Mit dem erfindungsgemäßen Verfahren können jegliche Verbindungen mit olefinischen Doppelbindungen im Molekül zu Epoxiden oxidiert werden. Pro Molekül können 1, 2 oder mehrere olefinischen Doppelbindungen enthalten sein. Die olefinischen Verbindungen können auch Heteroatome wie Sauerstoff, Schwefel und/oder Stickstoff enthalten. Die olefinischen Verbindungen können also reine Kohlenwasserstoffe, Ester, Alkohole, Ether, Säuren, Amine, Carbonylverbindungen oder auch polyfunktionelle Verbindungen sein, die vorzugsweise 2 bis 30 Kohlenstoffatome im Molekül aufweisen, besonders bevorzugt mindestens 3 Kohlenstoffatome. Das Verfahren ist insbesondere anwendbar für geradkettige, verzweigte oder ringförmige, substituierte oder unsubstituierte aliphatische olefinische Verbindungen oder olefinische Verbindungen mit einem Arylbestandteil im Molekül, vorzugsweise für olefinische Verbindungen mit 2 bis 30 Kohlenstoffatomen, besonders bevorzugt mit mindestens 3 Kohlenstoffatomen. Als Substituenten können Halogensubstituenten oder Sauerstoff, Schwefel oder Stickstoff enthaltende Substituenten enthalten sein.

### Ausführungsbeispiele

### Beispiel 1:

Epoxidierung von Amylen (2-Methyl-2-Buten) bei 300 °C und 500 mbar für verschiedene feed-Verhältnisse Amylen/O₃ von 1.43 bis 3.47.

Der Olefin-Gasstrom (4 Standard-Liter / min.) bestehend aus Amylen und N₂ wird auf 550°C vorgeheizt. Der O₃/NOₓ-Gasstrom (2 Standard-Liter / min.) bestehend aus 6.5 Vol.-% NO₂, 36 Vol.-% einer O₃/O₂ Mischung (aus Ozongenerator) und 57.5 Vol.-% N₂ wird ausgehend von Raumtemperatur über Düsen zum vorgeheizten Olefin-Gasstrom gegeben. Die Reaktionstemperatur beträgt 300°C. Nach Mischen beträgt der Ozon-Gehalt 0.7 Vol.-% und der Amylen-Gehalt 1.0 - 2.4 Vol.-%. Die Bulk-Verweilzeit in der Reaktionszone beträgt 4.8 ms.

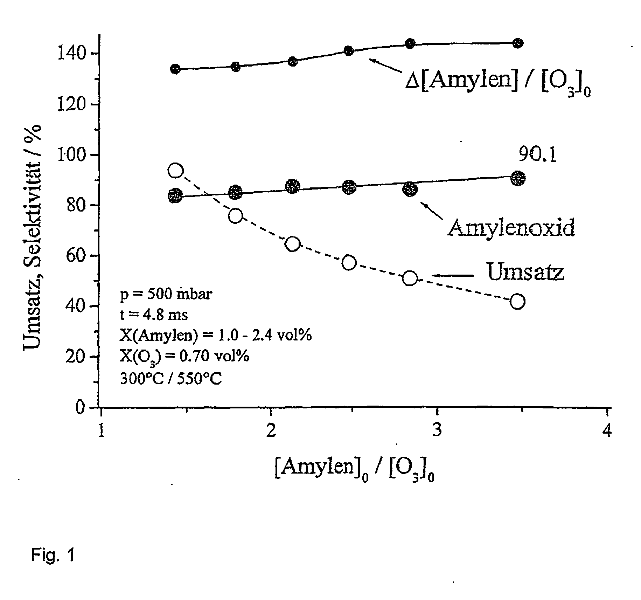

Als Nebenprodukte werden Acetaldehyd und Aceton gefunden. Die Ergebnisse sind in Fig. 1 dargestellt.

Die Parameter am Arbeitspunkt der höchsten Selektivität beim feld-verhältnis Amylen/O₃ ≐ 3.47 betragen:
Umsatz an Amylen: 41.3 %
Selektivität zum Amylenoxid: 90.1 mol %
umgesetztes Amylenleingesetztes O₃: 1.43 (molar)
Raum-Zeit-Ausbeute: 6240 g Amylenoxid / h / (Liter Reaktorvolumen),

### Beispiel 2:

Epoxidierung von TME (Tetramethylethylen) bei 200 °C und 500 mbar für verschiedene feed-Verhältnisse TME/O₃ von 1.43 bis 5.24

Der Olefin-Gasstrom (2 Standard-Liter / min.) bestehend aus TME und N₂ wird auf 320°C vorgeheizt. Der O₃/NOₓ-Gasstrom (1 Standard-Liter / min.) bestehend aus 6 Vol.-% NO₂ 25 Vol.-% einer O₃/O₂ Mischung (aus Ozongenerator) und 69 Vol.-% N₂ wird ausgehend von Raumtemperatur über Düsen zum vorgeheizten OlefinGasstrom gegeben. Die Reaktionstemperatur beträgt 200°C. Nach Mischen beträgt der Ozon-Gehalt 0.59 Vol.-% und der TME-Gehalt 0.84 - 3.1 Vol.-%. Die Bulk-Verweilzeit in der Reaktionszone beträgt 9.6 ms.

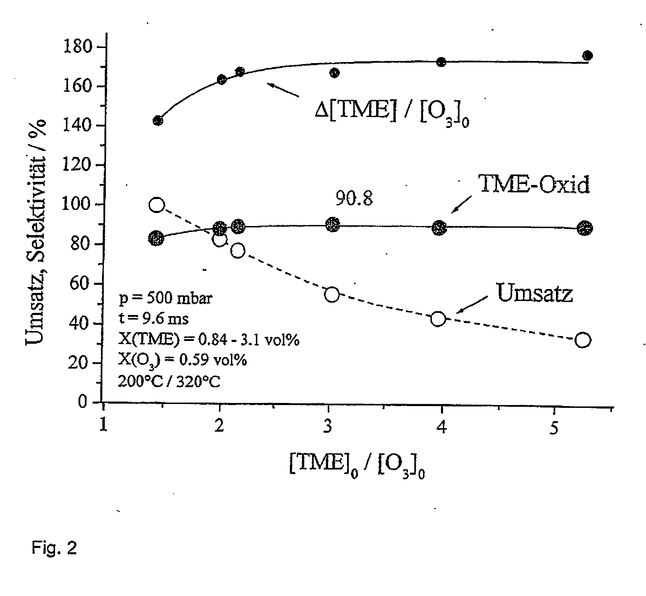

Als Nebenprodukte werden Aceton und Pinacolon gefunden. Die Ergebnisse sind in Fig. 2 dargestellt.

Die Parameter am Arbeitspunkt der höchsten Selektivität beim feed-Verhältnis TME/O₃ = 3.02 betragen:
Umsatz an TME: 55.6 %
Selektivität zum TME-Oxid: 90.8 Mol %
umgesetztes TAIIE/eingesetztes O₃: 1.68 (molar)
Raum-Zeit-Ausbeute: 3600 g TME-Oxid / h / (Liter Reaktorvolumen).

### Beispiel 3:

### Epoxidierung von Propylen bei 300 °C und 500 mbar

Der Olefin-Gasstrom (4 Standard-Liter / min.) bestehend aus Propylen und N₂ wird auf 550°C vorgeheizt. Der O₃/NOₓ-Gasstrom (2 Standard-Liter / min.) bestehend aus 2.25 Vol.-% NO₂, 10 Vol.-% einer O₃/O₂ Mischung (aus Ozongenerator) und 87.75 Vol.-% N₂ wird ausgehend von Raumtemperatur über Düsen zum vorgeheizten Olefin-Gasstrom gegeben. Die Reaktionstemperatur beträgt 300°C. Nach Mischen beträgt der Ozon-Gehalt 0.27 Vol.-% und der Propylen-Gehalt 5.6 Vol.-%. Die Bulk-Verweilzeit in der Reaktionszone beträgt 4.8 ms.

Als Nebenprodukte werden Formaldehyd und Acetaldehyd gefunden.

Die Parameter am Arbeitspunkt betragen:
Umsatz an Propylen: 4.6 %
Selektivität zum Propylenoxid: 81.3 Mol %
umgesetztes Propylen/eingesetztes O₃: 0.98 (polar)
Raum-Zeit-Ausbeute: 980 g Propylenoxid / h / (Liter Reaktorvolumen)

### Beispiel 4:

### Epoxidierung von TME (Tetramethylethylen) bei 300 °C und 1000 mbar für verschiedenen O₂-Gehalt im Reaktionsgas

Der Olefin-Gasstrom (4 Standard-Liter / min.) bestehend aus TME und N₂ wird auf 460°C vorgeheizt. Der O₃/NOₓ-Gasstrom (2 Standard-Liter / min.) bestehend aus jeweils 5 Vol.-% NO₂ und 25 Vol.-% einer O₃/O₂ Mischung (aus Ozongenerator) sowie 70 Vol.-% N₂ oder 45 Vol.-% N₂ und 25 Volt O₂ wird ausgehend von Raumtemperatur über Düsen zum vorgeheizten Olefin-Gasstrom gegeben. Die Reaktionstemperatur beträgt 300°C. Nach Mischen beträgt der Ozon-Gehalt 0.4 Vol.-% und der TME-Gehalt 1.62 Vol.-% oder 1.43 Vol.-% (bei höherem O₂-Gehalt). Der O₂-Gehalt beträgt entweder 8.3 Vol.-% oder 16.7 Vol.-% (bei Zumischen von 25 Vol.-% O₂ im O₃/NOₓ-Gasstrom). Die Bulk-Verweilzeit in der Reaktionszone beträgt 9.6 ms.

Als Nebenprodukte werden Aceton und Pinacolon gefunden.

Die Parameter am Arbeitspunkt bei einem O₂-Gehalt von 8.3 Vol.-% betragen: Umsatz an TME: 43.7 %
Selektivität zum TME-Oxid: 87.4 Mol %
umgesetztes TME/eingesetztes O₃: 1.78 (molar)
Raum-Zeit-Ausbeute: 4950 g TME-Oxid / h / (Liter Reaktorvolumen)

Die Parameter am Arbeitspunkt bei einem O₂-Gehalt von 16.7 Vol.-% betragen:
Umsatz an TME: 45.2 %
Selektivität zum TME-Oxid: 89.6 Mol %
umgesetztes TME/eingesetztes O₃: 1.64 (molar) Raum-Zeit-Ausbeute: 4650 g TME-Oxid / h / (Liter Reaktorvolumen).

In den Beispielen bedeuten:

| | |
|---|---|
| Verweilzeit | = Verweilzeit des Gasgemisches in der Reaktionszone des Strömungsreaktors |
| 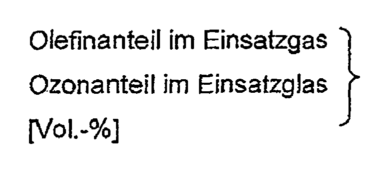 | = am Gesamtstrom in der Reaktionszone |
| Umsatz an Olefin [Mol %] | = Verhältnis von umgesetzten Molen an Olefin zu eingesetzten Molen an Olefin x 100 % |
| Selektivität zum Epoxid [Mol %] | = Verhältnis von gebildeten Molen an Epoxid zu umgesetzten Molen an Olefin x 100 % |
| umgesetztes Olefin/ eingesetztes O₃ (Δ [Olefin] / [O₃] ₀) | = Verhältnis von umgesetzten Molen an Olefin zu eingesetzten Molen an Ozon |

## Patentansprüche

1. Verfahren zur Herstellung von Epoxiden durch Oxidation von Olefinen in einer homogenen Gasphasenreaktion, indem das mittels Trägergas zugeführte Olefin in einem Strömungsreaktor mit einer Gasmischung aus Ozon und NO₂ und/oder NO als Oxidationsmittel ohne Verwendung eines Katalysators umgesetzt wird, wobei Ozon und NO₂ und/oder NO in einer dem Strömungsreaktor vorgeschalteten Mischkammer gemischt werden, **dadurch gekennzeichnet, dass**
das Olefin in der Reaktionszone des Strömungsreaktors bei einer Reaktionstemperatur von etwa 150 °C bis etwa 450 °C und einem Druck von 250 mbar bis 10 bar mit der Gasmischung des Oxidationsmittels umgesetzt wird, wobei der das Olefin enthaltende Trägergasstrom in einer Vorheizzone des Strömungsreaktors auf eine Temperatur von 250 °C bis 650 °C erhitzt wird, die Gasmischung des Oxidationsmittels aus der Mischkammer mit Umgebungstemperatur dem Olefin in der Reaktionszone des Strömungsreaktors turbulent zugemischt wird, so dass die Reaktionstemperatur beim Zumischen erreicht wird, und das Verhältnis zwischen Olefin-Gasstrom und Gasstrom des Oxidationsmittels von 5 : 1 bis 1 : 1 beträgt.

2. Verfahren nach Anspruch 1, **dadurch gekennzeichnet, dass** die Umsetzung im Strömungsreaktor bei 500 bis 2000 mbar, vorzugsweise bei mehr als 1.000 mbar, besonders bevorzugt bei atmosphärischem Druck, durchgeführt wird.

3. Verfahren nach Anspruch 1 oder 2, **dadurch gekennzeichnet, dass** der das Olefin enthaltende Trägergasstrom in der Vorheizzone des Strömungsreaktors auf eine Temperatur von 400 °C bis 550 °C vorgeheizt wird.

4. Verfahren nach einem der Ansprüche 1 bis 3, **dadurch gekennzeichnet, das** die Reaktionstemperatur in der Reaktionszone des Strömungsreaktors etwa 200 °C bis etwa 350 °C beträgt.

5. Verfahren nach einem der Ansprüche 1 bis 4, **dadurch gekennzeichnet, dass** das Verhältnis zwischen Olefingasstrom und Gasstrom des Oxidationsmittels von 4: 1 bis 2 : 1 beträgt.

6. Verfahren nach einem der Ansprüche 1 bis 5, **dadurch gekennzeichnet, dass** als Trägergas für das Olefin und für das Gasgemisch des Oxidationsmittels ein Inertgas, Sauerstoff oder Luft oder Gemische der genannten Gase eingesetzt werden, vorzugsweise Stickstoff.

7. Verfahren nach einem der Ansprüche 1 bis 6, **dadurch gekennzeichnet, dass** Ozon als Ozon/Sauerstoff-Gemisch eingesetzt wird.

## Claims

1. A method for producing epoxides by oxidizing olefins in a homogeneous gas-phase reaction by reacting the olefin supplied by means of a carrier gas within a flow reactor with a gas mixture of ozone and NO₂ and/or NO as an oxidant without using a catalyst, wherein ozone and NO₂ and/or NO are mixed in a mixing chamber arranged upstream the flow reactor.
**characterized in that**
the olefin is reacted in the reaction zone of the flow reactor at a reaction temperature of about 150°C to about 450°C and a pressure of 250 mbar to 10 bar with the gas mixture of the oxidant, wherein the carrier gas stream containing the olefin is heated to a temperature of 250°C to 650°C in a preheating zone of the flow reactor, the gas mixture of the oxidant from the mixing chamber having ambient temperature is admixed to the olefin in the reaction zone of the flow reactor by way of turbulence so that the reaction temperature is reached during the admixing, and the ratio between the olefin-gas stream and the gas stream of the oxidant is from 5:1 to 1:1.

2. The method according to claim 1, **characterized in that** the reaction in the flow reactor is performed at 500 to 2000 mbar, preferably at more than 1000 bar, particularly preferred at atmospheric pressure.

3. The method according to claim 1 or 2, **characterized in that** the carrier gas stream containing the olefin is preheated to a temperature of 400°C to 550°C in the preheating zone of the flow reactor.

4. The method according to any one of claims 1 to 3, **characterized in that** the reaction temperature in the reaction zone of the flow reactor is about 200°C to about 350°C.

5. The method according to any one of claims 1 to 4, **characterized in that** the ratio between the olefin-gas stream and the gas stream of the oxidant is from 4:1 to 2:1.

6. The method according to any one of claims 1 to 5, **characterized in that** an inert gas, oxygen or air or mixtures of the mentioned gases, preferably nitrogen, are used as a carrier gas for the olefin and for the gas mixture of the oxidant.

7. The method according to any one of claims 1 to 6, **characterized in that** ozone is used as an ozone/oxygen mixture.

## Revendications

1. Procédé de fabrication d'époxydes par oxydation d'oléfines dans une réaction homogène de phase gazeuse, consistant en ce que l'oléfine amenée par gaz vecteur est convertie dans un réacteur à écoulement avec un mélange gazeux constitué d'ozone et de NO₂ et/ou de NO comme agent oxydant sans utilisation d'un catalyseur, de l'ozone et du NO₂ et/ou du NO étant mélangés dans une chambre de mélange placée en aval du réacteur à écoulement,
**caractérisé en ce que**
l'oléfine est convertie avec le mélange gazeux de l'agent oxydant dans la zone de réaction du réacteur à écoulement à une température de réaction d'environ 150 °C à environ 450 °C et une pression de 250 mbars à 10 bars, le flux de gaz vecteur contenant l'oléfine étant chauffé à une température de 250 °C à 650 °C dans une zone de préchauffage du réacteur à écoulement, le mélange gazeux de l'agent oxydant provenant de la chambre de mélange à température ambiante étant adjoint de manière turbulente à l'oléfine dans la zone de réaction du réacteur à écoulement de façon que la température de réaction soit atteinte lors de l'adjonction, et le rapport entre le flux de gaz de l'oléfine et le flux de gaz de l'agent oxydant étant de 5:1 à 1:1.

2. Procédé selon la revendication 1, **caractérisé en ce que** la conversion dans le réacteur à écoulement est effectuée à une pression comprise entre 500 et 2 000 mbars, préférentiellement à plus de 1 000 mbars, et plus préférentiellement à la pression atmosphérique.

3. Procédé selon la revendication 1 ou 2, **caractérisé en ce que** le flux de gaz vecteur contenant l'oléfine est préchauffé à une température de 400 °C à 550 °C dans la zone de préchauffage du réacteur d'écoulement.

4. Procédé selon l'une des revendications 1 à 3, **caractérisé en ce que** la température de réaction dans la zone de réaction du réacteur à écoulement est d'environ 200 °C à environ 350 °C.

5. Procédé selon l'une des revendications 1 à 4, **caractérisé en ce que** le rapport entre le flux de gaz de l'oléfine et le flux de gaz de l'agent oxydant est de 4:1 à 2:1.

6. Procédé selon l'une des revendications 1 à 5, **caractérisé en ce qu'**un gaz inerte, de l'oxygène ou de l'air ou des mélanges des gaz cités, et préférentiellement de l'azote, sont utilisés comme gaz vecteur pour l'oléfine et pour le mélange gazeux de l'agent oxydant.

7. Procédé selon l'une des revendications 1 à 6, **caractérisé en ce que** de l'ozone est utilisé comme mélange ozone/oxygène.
